Europäisches Patentamt

(19) European Patent Office

Office européen des brevets

(11) Publication number: **0 047 474**
A1

## (12) EUROPEAN PATENT APPLICATION

(21) Application number: **81106831.1**

(22) Date of filing: **01.09.81**

(51) Int. Cl.³: **C 07 D 301/02**

(30) Priority: **04.09.80 US 184066**

(43) Date of publication of application: **17.03.82**
**Bulletin 82/11**

(84) Designated Contracting States: **BE DE FR GB IT NL**

(71) Applicant: **UNION CARBIDE CORPORATION, 270, Park Avenue, New York, N.Y. 10017 (US)**

(72) Inventor: **McMullen, Charles Henry, 44 Sunrise Avenue, Katonah (10536) New York (US)**

(74) Representative: **Wuesthoff, Franz, Dr.-Ing. et al, Patentanwälte Wuesthoff -v. Pechmann-Behrens-Goetz Schweigerstrasse 2, D-8000 München 90 (DE)**

(54) **Process for the preparation of epoxides from alkylene carbonates.**

(57) A homogeneous catalytic process for converting an alkylene carbonate to the corresponding epoxide wherein a liquid phase comprising said alkylene carbonate and a catalyst is heated at a temperature of from about 100°C to about 250°C to form a product mixture comprising said epoxide and carbon dioxide. The improvement comprises introducing a continuous stream of inert gas into the liquid phase so as to remove therefrom at least a portion of the product epoxide formed by said process thereby minimizing the formation of undesired aldehyde and/or ketone by-products.

2

This invention relates to a process for converting alkylene carbonates to their corresponding epoxides. More particularly, this invention is concerned with a process for generating an alkylene carbonate such as ethylene oxide from the corresponding alkylene carbonate with a minimum formation of undesired aldehyde and/or ketone byproducts.

Processes for the efficient conversion of alkylene carbonates, particularly ethylene and propylene carbonates, to the corresponding epoxides (namely, ethylene oxide and propylene oxide) have heretofore been the focus of relatively limited study in the prior art. Processes to synthesize the alkylene carbonate starting material from an alkylene oxide and carbon dioxide are more common in the art and are amply described in the patent literature. U. S. Patent Nos. 2,773,070; 2,873,282; 2,907,771; and 2,994,704 are descriptive of such carbonate-forming process. Belgian patent No. 872,960 corresponding to U. S. Patent application Serial No. 863,354, filed December 22, 1977, assigned to Union Carbide Corporation is directed to a particularly efficient process for alkylene carbonate formation which is characterized by alkylene carbonate efficiencies above 99% and an epoxide conversion of greater than 99.5%. In view of the relatively advanced state of the art of alkylene carbonate formation as reflected by the patents disclosed above, a process which efficiently converts an alkylene carbonate, such as ethylene carbonate, to ethylene oxide could be advantageously combined with an efficient alkylene carbonate forming process so as to allow ethylene carbonate to be used as an economical, safe and non-explosive medium of transportation for ethylene oxide. The ethylene oxide could thus be efficiently generated from ethylene carbonate upon demand and thereby avoid the inherent hazards of shipping and storing the flammable oxide prior to use. Moreover, in instances where the reactant alkylene carbonate is produced from non-epoxide starting materials, such carbonate would represent a particularly desirable source for generating alkylene oxide.

A major source of inefficiency in the conversion of an alkylene carbonate to an alkylene oxide and carbon dioxide is the formation of aldehydes and/or ketones as byproducts of the reaction. For processes where the alkylene carbonate reactant is, for example, ethylene carbonate, acetaldehyde is generally formed as an undesired byproduct. Similarly, the conversion of propylene carbonate usually results in the undesired formation of propionaldehyde and/or acetone; the particular aldehyde and/or ketone thus produced being dependent upon the choice of alkylene carbonate reactant. The presence of such aldehyde and ketone byproducts is deemed unacceptable in commercial grade alkylene oxides because they adversely affect the products ultimately formed by the catalyzed reactions of such alkylene oxides. Moreover, the separation of such aldehydes and ketones from alkylene oxide is troublesome and costly. For example, the separation of acetaldehyde from ethylene oxide is a relatively difficult operation inasmuch as both materials are liquids having relatively similar volatilities. As a consequence, separation is usually effected in commercial operation by a relatively elaborate fractional distillation.

## SUMMARY OF THE INVENTION

The invention describes a homogeneous process for converting an alkylene carbonate to the corresponding epoxide wherein a liquid phase containing the alkylene carbonate and a catalyst is heated to form a product mixture comprising predominantly alkylene oxide and carbon dioxide. The process is characterized by the improvement wherein a stream of inert gas is continuously introduced into the liquid phase so as to remove from said liquid phase at least a portion of the product epoxide formed by the process.

The present invention is predicated on the discovery that the continuous sparging of the homogeneous liquid phase with a stream of inert gas substantially reduces the formation of undesired aldehyde and/or ketone byproducts. Although the mechanism by which such undesired byproduct formation is reduced is not fully understood, it is believed that by continuously removing alkylene oxide from the liquid phase with a stream of inert gas and thereby minimizing its residence time in the liquid phase, the availability of such alkylene oxide to interact with alkylene carbonate and the homogeneous catalyst is correspondingly reduced thereby preventing the undesired side reactions which result in the formation of aldehyde and ketone byproducts.

The term "inert gas" as used throughout the specification and claims refers to any gas which when introduced into the liquid phase described herein does not react to form products other than alkylene carbonate, alkylene oxide or carbon dioxide. Thus, for example, oxygen is not considered an inert gas for purposes of the

5

0047474

invention because it reacts with the alkylene carbonate to form additional byproducts. Carbon dioxide, on the other hand, while it may affect the kinetics of the conversion reaction of alkylene carbonate to epoxide, does not react in the liquid phase to form products other than alkylene carbonate or alkylene oxide. Accordingly, the inert gases contemplated by the invention are most conveniently nitrogen, helium and carbon dioxide.

## DETAILED DESCRIPTION OF THE INVENTION

The preferred alkylene carbonates contemplated by the invention are ethylene carbonate and propylene carbonate, such carbonates being reacted by the process of the invention to produce ethylene oxide and propylene oxide, respectively. Other alkylene carbonates may, however, be also advantageously used in accordance with the invention to produce the corresponding epoxide.

The invention contemplates the utilization of known catalysts for the conversion of alkylene carbonate to alkylene oxide. Thus, the catalyst may be any of those which are specifically defined by the prior art. For example, U. S. Patent No. 4,069,234 describes the use of phosphonium halide catalysts for the formation of vicinal epoxides from carbonates. Alkali metal halides, such as described in Shapiro et al., J. Org. Chem. U.S.S.R., 5(2), p. 222 (1969), are generally preferred for the reaction.

The catalysts may be employed in amounts ranging from as little as 0.001 moles/liter in the liquid phase, based on the alkylene carbonate reactant, to about 0.1 mole/liter. Generally a concentration of from about 0.01 to 0.05 mole/liter is desirable.

- 5 -

The primary materials contained in the homogeneous liquid phase in the conversion of alkylene carbonate to alkylene oxide is the alkylene carbonate reactant and a catalyst. A solvent is generally not necessary but if desired, an inert solvent such as tetraglyme or tetralin may be added to the liquid phase. This is particularly desirable in a continuous reaction system as a means of maintaining the catalyst in solution during catalyst recycl

The inert gas stream is desirably passed through the liquid body in a manner so as to achieve a high degree of gas dispersion throughout the liquid mixture and thereby maximize the amount of product alkylene glycol removed by the gas stream. A variety of gas distribution techniques may be employed. For example, the gas stream may be introduced through a sparger into the liquid phase mixture containing the product alkylene glycol. The sparger may be in the form of a ring having a multiplicity of orifices at the bottom of the vessel which contains the liquid mixture or more simply, a tube having a fritted disc at its end. Stirring means may be provided within the vessel to insure good distribution of the gas through the liquid phase. Similarly, a baffle-type arrangement may be used to direct the gas flow as it rises from the bottom of the liquid phase to the top.

The process temperature of the reaction is generally maintained between 100°C and 250°C. The minimum temperature of the reaction is that temperature at which the catalytic decomposition of alkylene carbonate to alkylene oxide and carbon dioxide will occur. At temperatures below 100°C the rate of reaction of such

- 6 -

carbonate decomposition is generally too low for practical consideration. Temperatures above 250°C are generally avoided so as to minimize the rate of reaction to undesired aldehyde and/or ketone byproducts. For most effective operation of the process, a temperature range of from about 170°C to about 220°C is preferred.

The process of the invention is conveniently carried out at atmospheric pressure. However, operation of the process at sub-atmospheric pressure is particularly desirable for purposes of reducing aldehyde and/or ketone byproduct, such process operation being more fully described in copending application Serial No.          , filed on even date herewith (D-12,530).

DESCRIPTION OF TEST PROCEDURE

The test reactor used in the following examples was a 75 ml carius tube sealed at the top with a metal cap fitted with a gas purge tube having a fritted disc at its base for introducing a gas stream into the liquid phase and having ports for adding catalyst and alkylene carbonate to the reactor and sampling the effluent gas produced by the reaction. The test reactor was immersed in an oil bath to maintain a constant reaction temperature. The reactor was used in a continuous mode in which liquid alkylene carbonate was fed at a rate sufficient to maintain a constant liquid level. The effluent vapors leaving the reactor were passed into a 316 stainless steel manifold prior to being sampled and analyzed for ethylene oxide and acetaldehyde in a Perkin-Elmer model 990 gas

8                                    0047474

chromatograph with a thermal conductivity detector. The
effluent or off-gas of the reactor was discharged from the
manifold through a flowmeter.

Doubly-distilled ethylene carbonate was
distilled from calcium hydride prior to its use in the
examples below.  A fraction having a boiling point of
from 128-129°C/124mm was collected.  The ethylene carbonate thus recovered was weighed and introduced into the
reactor which was maintained at the indicated reaction
temperature by the oil bath.

Referring to Table I, Example 1 was a control
example in which a large amount of acetaldehyde (8400 ppm)
byproduct was formed.  In Examples 2 and 3, the use of
an inert gas sparge of helium and carbon dioxide, respectively, under near identical reaction conditions
relative to Example 1 resulted in a greater than 70% reduction in acetaldehyde formation; specifically, the forma-
tion of 2600 and 2000 ppm, respectively.

The effect of minimized aldehyde formation
attendant to an inert gas sparge of the liquid phase was
also noted in Examples 4 and 5 which used different
catalysts than that used in Example 1, Example 5 being
also operated at a higher temperature than Example 1.

## TABLE I

### CONVERSION OF ETHYLENE CARBONATE (EC) TO ETHYLENE OXIDE AND $CO_2$ CATALYZED BY ALKALI METAL SALTS

| Example | Catalyst | Catalyst Concentration (Moles/Liter) | Reaction Temp. (°C) | Inert Gas and Flow Rate | Reaction Time (hr) | Rate of EC Conversion (Mole/Liter) (hr) | Acetaldehyde[a] (ppm) |
|---|---|---|---|---|---|---|---|
| 1 | NaBr | 0.066 | 180 | - | 6.0 | 1.4 | 8400 |
| 2 | NaBr | 0.071 | 180 | He (400)[b] | 5.0 | 2.9 | 2600 |
| 3 | NaBr | 0.066 | 180 | $CO_2$ (350)[b] | 10.0 | 1.4 | 2000 |
| 4 | LiBr | 0.018 | 180 | $CO_2$ (400)[b] | 9.5 | 1.0 | 2000 |
| 5 | KBr | 0.070 | 200 | $CO_2$ (400)[b] | - | 1.7 | 1500 |

Footnote: [a] The ppm of acetaldehyde are relative to ethylene oxide.

[b] Purge rate in ml/min.

0047474
12,903

PATENTANWÄLTE

WUESTHOFF-v. PECHMANN-BEHRENS-GOETZ

EUROPEAN PATENT ATTORNEYS

DR.-ING. **0047474**
DR. PHIL. FREDA WUISTHOFF (1927-1956,
DIPL.-INC. GERHARD PUIS (1952-1971)
DIPL.-CHEM DR. E. FREIHERR VON PECHMANN
DR.-ING. DIFTER BEHRENS
DIPL.-ING.; DIPL.-WIRTSCH.-ING. RUPERT GOETZ

EP-55 095          - 10 -

D-8000 MÜNCHEN 90
SCHWEIGERSTRASSE 2

TELEFON: (089) 66 20 51
TELEGRAMM: PROTECTPATENT
TELEX: 5 24 070

Patent    Claims

1.  In a homogeneous catalytic process for converting an alkylene carbonate to the corresponding epoxide wherein a liquid phase comprising said alkylene carbonate and a catalyst is heated at a temperature of from about 100°C to about 250°C to form a product mixture comprising said epoxide and carbon dioxide, the improvement for minimizing the formation of undesired aldehyde and/or ketone byproducts which comprises introducing a continuous stream of inert gas into said liquid phase so as to remove therefrom at least a portion of the product epoxide formed by said process.

2.  The process of claim 1 wherein said reaction temperature is from about 170°C to about 220°C.

3.  The process of claim 1 wherein said inert gas is carbon dioxide.

0047474

4.    The process of claim 1 wherein said inert gas is nitrogen.

5.    The process of claim 1 wherein said inert gas is helium.

European Patent Office

**EUROPEAN SEARCH REPORT**

| DOCUMENTS CONSIDERED TO·BE RELEVANT | | | CLASSIFICATION OF THE APPLICATION (Int. Cl.³) |
|---|---|---|---|
| Category | Citation of document with indication, where appropriate, of relevant passages | Relevant to claim | |
| X | DE - C - 845 937 (BADISCHE ANILIN- & SODA-FABRIK)<br>* claims 1, 2; example 1 *<br>-- | 1-4 | C 07 D 301/02 |
| D | US - A - 4 069 234 (Y. WU)<br>* claims 1, 2 *<br>-- | 1 | |
| A | US - A - 2 851 469 (J.M.A. TESTARD)<br>-- | | **TECHNICAL FIELDS SEARCHED** (Int. Cl.³) |
| A | DE - A1 - 2 855 232 (UNION CARBIDE CORP.) | | |
| D | & BE - A - 872 960<br>---- | | C 07 D 301/02 |

CATEGORY OF CITED DOCUMENTS

X: particularly relevant
A: technological background
O: non-written disclosure .
P: intermediate document
T: theory or principle underlying the invention
E: conflicting application
D: document cited in the application
L: citation for other reasons

&: member of the same patent family, · corresponding document

| | The present search report has been drawn up for all claims | | |
|---|---|---|---|
| Place of search<br>Berlin | Date of completion of the search<br>12-11-1981 | Examiner<br>FROELICH | |

EPO Form 1503.1  06.78